# EUROPEAN PATENT APPLICATION

(11) **EP 4 193 919 A1**
(43) Date of publication of application: **14.06.2023**
(21) Application number: 21214782.1
(22) Date of filing: 15.12.2021
(51) Int. Cl.: A61B 5/055, G01R 33/28, F21V 8/00, G09F 13/18

(54) **PATTERNED EDGE-ILLUMINATED DISPLAY FOR USE IN EXAMINATION OF VISUAL EVOKED POTENTIALS WITH SIMULTANEOUS MR IMAGING**

(30) Priority: 10.12.2021 PL 43980321
(71) Applicant: Uniwersytet w Bialymstoku, 15-328 Bialystok (PL); Uniwersytet Medyczny w Bialymstoku, 15-089 Bialystok (PL)
(72) Inventor: Szymanski, Krzysztof, Bialystok (PL); Labieniec, Lukasz, Bialystok (PL); Lisowski, Lukasz, Wasilkow (PL); Mojsak, Malgorzata Natalia, Bialystok (PL)
(74) Representative: Wlasienko, Jozef

(57) **Abstract**

The object of the invention relates to a patterned edge-illuminated display (100) for use in the examination of visual evoked potentials with simultaneous MR imaging, comprising at least two plates, a first plate (110) and a second plate (120), wherein the first plate (110) and the second plate (120) are transparent, internally reflective light transmitting plates, each of the first plate (110) and the second plate (120) has two major flat surfaces parallel to each other, and edges, a first light source (131) and a second light source (132) distributed along and optically coupled to first plate edges (113) and second plate edges (123), respectively, a light source controlling means (140) and a first plate pattern (111) applied to at least the first major surface of the first plate (110) and a second plate pattern (121) applied to at least the first major surface of the second plate (120), which are configured to extract light from the first plate (110) and the second plate (120) respectively. The display further comprises an opaque background board (150), wherein the first plate (110) and the second plate (120) are stacked on the opaque background board (150), all with edges juxtapositioned to a respective edges of each other forming a display board (105). The first plate pattern (111) is complementary to the second plate pattern (121) and the first light source (131) is distributed along all of the first plate edges (113) and the second light source (132) is distributed along all of the second plate edges (123) and the light source controlling means (140) alternately illuminates the first light source (131) and the second light source (132) such that the light is supplied only to all of the first plate edges (113) or to all of the second plate edges (123) at a time.

## Description

### FIELD OF THE INVENTION

The object of the invention relates to a patterned edge-illuminated display for use in the examination of visual evoked potentials with simultaneous MR imaging (also abbreviated as MRI). The display as such may serve alone as a tool for the examination of visual evoked potentials, however due to structural features that make the display substantially unsusceptible to electromagnetic fields, the display is especially suited to use in the examination of visual evoked potentials with simultaneous MR imaging. The object of the invention also relates to a system comprising the display, which can facilitate use of the display in the examination of visual evoked potentials, also with simultaneous MR imaging.

### BACKGROUND OF THE INVENTION

Visual evoked potentials (VEP) involve electrical activity on the scalp resulting from a visual stimulus and macroscopic electrical response of the surface layer of the brain underneath. VEP can be used to monitor the state of a visual pathway including the retina, the optic nerve, the optic chiasm, optic radiations, and the occipital cortex. The first VEP signals were registered in the forties of the 20^{th} century [Nor15].

The VEP signal is the time dependence of the potential difference with time locked to the stimulus onset. In a typical pattern-reversal VEP signal registered in occipital regions, there are two critical parameters: amplitude and latency. Signal of a healthy patient consists of the minimum with latency about 75 ms (N1), the maximum with latency about 100 ms (PI) and the minimum with latency about 135 ms (N2). The amplitudes represent the amount of information received by the visual cortex, whereas the latencies indicate how long the electrical signal took to travel from the retina to the visual cortex. Anomalies in the VEP signals are used for the detection of physiological dysfunctions of the optic pathway. In particular the VEP is used in Leber neuropathy, toxic neuropathy, deficiency neuropathy, glaucoma, idiopathic intracranial hypertension, idiopathic optic neuritis without systemic disease (recurrent isolated optic neuritis, chronic relapsing inflammatory optic neuropathy, solitary isolated optic neuritis) and diagnostic investigations of systematic diseases (connective tissue disease, granulomatous disease) [Too14], [Luo19], [Mar20]. With the help of VEP potentials during neurosurgical procedures within the visual pathway, the surgeon is able to remove a tumour with great accuracy without damaging the function of the optic nerve. By using MRI scans, optic nerve diseases can be diagnosed before the patient develops the full spectrum of symptoms. Therefore, earlier pharmacological or surgical treatment can be applied, achieving better therapeutic effects [Hen05] [Arr13].

Since the triggering time of the VEP signal to the visual stimulus onset is in the millisecond range, the VEP technique has a high temporal and relatively low spatial resolution. In contrast, functional magnetic resonance imaging (fMRI) serves a poor temporal and high (in the millimetre range) spatial resolution. Combination of these two techniques in a simultaneous mode has an advantage of access to both physiological correlates and neural processes, allowing exploration of the human brain function at high both temporal and spatial resolution. In first trials of the application of this type of combination separate VEP and fMRI experiments were performed [Kas01], [Hen05]. Later on it was demonstrated that simultaneous continuous VEP-fMRI recordings are feasible [Bec05]. The event-related functional magnetic resonance imaging (ERfMRI) has emerged as a technique that is useful in the investigations of brain hemodynamics elicited by brief, sparsely spaced stimuli. Therefore, as demonstrated above, convenient visual stimuli with well controlled time locking is important for neuroscience, ophthalmology and eye surgery.

In clinical practice VEP is measured as potential difference between two electrodes, one is placed 2.5 cm above the inion on the midline, the second is placed above a nasion. Typical pattern-reversal VEP signal refers to averaged time-locked responses to the visual stimuli, which is usually a checkboard in which white fields are replaced by black ones and black ones by white ones with a repetition rate of about 2 per second. The angular size of the checkboard during a medical examination, the reversal rate, the contrast and the luminance of the white and black fields are standardized [Odo16]. However, the amplitude of VEP signal is in the range of a few millivolts and the amplitude of statistical noise is usually ten times higher; therefore many repetitions and averaging have to be performed to filter out the noise.

As clearly argued above, a visual stimulus is of a great importance and must demonstrate required display reliability and dynamic characteristics. Precise time locking has to be performed with a precision of more than the tenths of milliseconds. In order to achieve these visual stimulus performances, various types of computer screens have been used.

Computer screens can serve as standard VEP technique computer displays located at the appropriate distance from the eyes. But for the simultaneous usage with fMRI acquisition, one must consider an influence of fMRI system electromagnetic fields on display, ranging from visual artefact generation to a complete damage of a display, and the influence of computer screen on fMR images by affecting signal acquisition.

### STATE OF THE ART

Many attempts have been made to solve the above-mentioned problem of the electromagnetic field interaction with a visual stimulus and visual stimulus electronics influence on fMRI signal acquisition, and they can be divided into two major categories:
- construction of visual stimuli devices which are unsusceptible to electromagnetic fields of the fMRI systems, and
- construction of shielding systems for some parts of the visual stimuli devices.

One example of a shielding system that was applied in order to protect electronic elements is presented in the patent application USOO5864331A wherein a display panel and associated electronics are mounted close to the MRI device and enclosed within a Faraday shield.

Also EP 1 887 932 B1 discloses a MRI device with a display means, wherein the display means comprises a micro display chip inside the Faraday cage.

US4613820A discloses a radio-frequency shielded room for a nuclear magnetic resonance imaging system which has at least one electrically-conductive shield wall substantially enclosing a predetermined volume adjacent to one end of the bore formed through an imaging magnet within which bore the imaging process is carried out.

The patent application DE10343721A1 discloses a visual stimulus sent to the patient eye by the endoscopic system. The endoscopic system is changeable to allow an enlargement and/or a correction of the illustration. An image transfer system with electric or electronic components are protected by the electromagnetic radiation shielding.

Another solution wherein both shielding and elements unsusceptible to electromagnetic fields were used is presented in [Huk02] wherein visual stimuli appear on a flat-panel display (multisynch LCD 2000; NEC, Itasca, IL) placed in a Faraday box with an electrically conductive glass front, positioned near the subjects' feet. The display is viewed by the subject through binoculars with a pair of angled mirrors attached just beyond the two objective lenses.

As far as the systems unsusceptible to magnetic fields are concerned, which may be positioned within the magnetic field of an MRI bore, one example is provided by US005877732A, which discloses a video and audio system within the magnetic field of a bore of an MRI scanner to provide video images via a relay lens system and corresponding audible sound via a non-magnetic path to an MRI patient. The invention provides non-magnetic video sources and audio speakers for generating the images and sound, respectively, and includes a first non-magnetic shielded generating mechanism positioned within the magnetic field of the bore for providing a video image.

Another example of a display that may be located in a bore of an MRI scanner is a display unit based on organic light-emitting diodes (OLED) which demonstrated its performance and compatibility with the MRI scanners [Ko18].

Another strategy of delivering visual stimuli is to use optical fibres adapted to custom-made goggles placed over the subject's field of vision. The stimuli may consist of flashes of white light with an appropriate intensity and duration as presented in [Arr13] and the system may be used together with Cambridge Research System and light-generator box.

In the known technical approach, the black and white checkerboard pattern was rear-projected by a colour LCD projector, through a collimating lens onto an acrylic screen that can also be located inside the MRI magnet, immediately below the subject's jaw. The visual stimulus was then transferred by a mirror interposed between and oriented approximately 45° to the screen and the subject's line of sight [Bon01].

As demonstrated above, shielding elements introduce structural complexity to the MRI systems. On the other hand, display means that are not sensitive to electromagnetic fields also require advanced technical adjustments to be employed within the MRI systems. Therefore, there is a need to look for simple and reliable solutions that would provide visual stimuli for use both with or without electromagnetic fields.

Therefore, the aim of the present invention is to provide a visual stimuli display, the performance of which is not affected by electromagnetic fields, that is less structurally complex, and thus easy to maintain, apply and manufacture, but at the same time demonstrates required display reliability and dynamic characteristics, as well as a precise time locking.

The above mentioned problems are solved and above mentioned objects are achieved by the present invention.

### SUMMARY OF THE INVENTION

The invention provides a patterned edge-illuminated display for use in the examination of visual evoked potentials with simultaneous MRI acquisition, comprising:
at least two plates, a first plate and a second plate, wherein the first plate and the second plate are transparent, internally reflective light transmitting plates, each of the first plate and the second plate has two flat major surfaces parallel to each other, and edges;
a first light source and a second light source distributed along and optically coupled to first plate edges and second plate edges, respectively;
a light source controlling means;
a first plate pattern applied to at least the first major surface of the first plate and a second plate pattern applied to at least the first major surface of the second plate, which are configured to extract light from the first plate and the second plate, respectively;
characterised in that
the display further comprises an opaque background board;
wherein the first plate and the second plate are stacked on the opaque background board, all with edges juxtapositioned to respective edges of each other forming a display board;
the first plate pattern is complementary to the second plate pattern; and
the first light source is distributed along all of the first plate edges and the second light source is distributed along all of the second plate edges, and the light source controlling means alternately illuminates the first light source and the second light source such that the light is supplied only to all of the first plate edges or to all of the second plate edges at a time.

Preferably, the first plate pattern and the second plate pattern are formed by tarnished, engraved parts of the first major surface of the first plate and the first major surface of the second plate.

Preferably, the first plate pattern and the second plate pattern are checkerboard patterns.

Preferably, a visual focusing element is arranged in the middle of the first plate and the second plate.

Preferably, the first plate and the second plate are made from colourless material.

Preferably, the first and the second light sources, the opaque background board and the visual focusing element have colour contrast between each other.

Preferably, the first plate, the second plate and the background screen are rectangular with the aspect ratio 4:3.

Preferably, the light source controlling means comprises a triggering system which triggers operation of the first light source and/or the second light source and provides the first light source and/or the second light source delay adjustment.

Preferably, the light source controlling means alternately illuminates the first light source and the second light source using rectangular signal of 2Hz frequency.

Preferably, the light source controlling means comprises a brightness adjustment system which adjust separately the brightness of the first light source distributed along and optically coupled to the first plate edges and the second light source distributed along and optically coupled to the second plate edges.

Preferably, the first plate pattern and the second plate pattern are checkerboard patterns having the dimensions of 40 cm by 30 cm divided into 20 by 14 rectangular fields or the dimensions of 39 cm by 29 cm divided into 78 by 58 rectangular fields.

Preferably, the visual focusing element is a dot of 7 mm in diameter positioned at corners of the four rectangular fields of the checkerboard pattern, which are located in the middle of the first plate and the second plate.

The invention also provides a system for use in the examination of visual evoked potentials with simultaneous MRI acquisition, characterised in that it comprises:
- a patterned edge-illuminated display according to the invention;
- reflecting means for suppling an image from the display screen to the patient's eye;
- system control means.

### DETAILED DESCRIPTION OF THE INVENTION

The invention provides a patterned edge-illuminated display for use in the examination of visual evoked potentials with simultaneous MRI acquisition. The display according to the invention can also be used as a stand-alone element for use in the examination of visual evoked potentials. However, the display according to the invention, due to its structural features that are essentially unsusceptible to electromagnetic fields, is designed for usage especially with simultaneous MR imaging.

The display according to the invention can be described as consisting of a unit unsusceptible to electromagnetic fields and a unit that is sensitive to electromagnetic fields. The unit that is unsusceptible to electromagnetic fields is a display screen and the unit that is sensitive to electromagnetic fields is a light source controlling means. The two units are connected by a cable that must as such be also unsusceptible to the electromagnetic fields, preferably a screened cable. The cable therefore constitutes a part of the display screen and/or the light source controlling means. This should be understood that the cable as such is an example of a connecting means within any device, herein the display, so for that reason any means that put the display screen in communication with the light controlling means can be used according to the invention.

The display screen comprises at least two display boards, i.e. at least the first display board and the second display board and an opaque background board.

In general, the display board comprises a plate which is a transparent, internally reflective light transmitting plate, and a light source. It means that the first display board comprises the first plate and the first light source and the second display board comprises the second plate and the second light source.

The first plate and the second plate are transparent, internally reflective light transmitting plates, each of the first plate and the second plate has two flat major surfaces parallel to each other, and edges. The plates, i.e. the first plate and the second plate can be any transparent, internally reflective light transmitting plates. Preferably, the plates are made of the same material. The material for the plates should be highly transparent, preferably colourless, and should demonstrate good workability. Therefore, the plates are preferably made of PMMA (poly(methyl methacrylate), polycarbonate or glass. However, other materials can also be used for that purpose and the person skilled in the art will select an appropriate material without undue burden.

As defined above, at least two display boards and the opaque background board form the main display element which is the display screen. The boards are preferably of the same size so they form a pile with all respective edges adjusted to each other. In other words, they preferably form at least three-layer display screen wherein the opaque background board forms the background for a pattern to be displayed on the display boards.

The first plate pattern is applied to at least the first major surface of the first plate and the second plate pattern is applied to at least the first major surface of the second plate. Since the plates are internally reflecting plates, they act as a light guide, thus the light introduced on one edge is guided in-between the plate major surfaces, unless the light arrives to the part of the plate major surface where the pattern, which is configured to extract the light from the plate, is applied.

The pattern is applied on a major surface of the plate, i.e. the first plate pattern is applied to at least the first major surface of the first plate and a second plate pattern is applied to at least the first major surface of the second plate, which are configured to extract the light from the first plate and the second plate, respectively. Here, the first major surface means the surface of the plate facing away from the background board.

The first pattern must be complementary to the second pattern so when the first plate overlays the second plate, the first pattern and the second pattern form substantially and visually a uniform plane. Then, when the light is provided to only the first plate, the first pattern shines and is visible, and after switching, when the light is provided to only the second plate, the second pattern shines and is visible. By switching the light supply from the first plate to the second plate and again to the first plate, and so on, an alternating pattern effect is achieved which is required for visual stimuli for the examination of visual evoked potentials.

Preferably, the patterns are checkerboard patterns, which means that alternated dark and lighted rectangular fields will be displayed on the display screen when the light is supplied to the first plate or to the second plate, at a time. Preferably, the pattern is in accordance with ISCEV standards [http://www.iscev.org] and may be checkerboard patterns having the dimensions of : 40 cm by 30 cm divided into 20 by 14 rectangular fields or 39 cm by 29 cm divided into 78 by 58 rectangular fields.

The first pattern and the second pattern can be applied by any technique known to the person skilled in the art and suitable to alter the surface of the plate in order to extract the light from its major surface, i.e. the first major surface of the first plate and the first major surface of the second plate, wherein the first surface of the first plate and the first surface of the second plate are major surfaces of the plates (the first plate and the second plate). Preferably, the pattern is engraved and as a result part of the plate main surface is tarnished. Other pattern providing techniques and methods can also be used for that purpose and the person skilled in the art will select an appropriate method to provide the pattern without undue burden.

The patterns preferably covers substantially whole first major surface of the first plate and the first major surface of the second plate. The first plate, the second plate and the background screen are therefore also rectangular with the aspect ratio 4:3, wherein the aspect is the ratio of a width to a height of an object.

The light source is distributed along all of the first plate edges and all of the second plate edges. More precisely, the first light source is distributed along all of the first plate edges and the second light source is distributed along all of the second plate edges in order to provide a uniform light distribution within the plates. The first light source and the second light source are illuminated by the light source controlling means alternately, such that the light is supplied only to all of the first plate edges or to all of the second plate edges at a time.

The light source can be of any kind suitable to be optically coupled to the edges of the plate. Preferably, the light source comprises LEDs. Also preferably, the light source comprises optical fibres powered by a laser, for example 10 W RGB laser module in the visual range of 470-700 nm equipped with fibre optic splitter and multimode optical fibres.

Preferably, the display comprises a visual focusing element arranged in the middle of the first plate and in the middle of the second plate. The visual focusing element is preferably a dot. The visual focusing element may be of any size suitable for focusing an eyesight but the diameter should not exceed the size of two rectangular fields, so it can be for example, approximately 7 mm in diameter. Preferably, the visual focusing element is positioned at corners of the four rectangular fields of the checkerboard pattern, which are located in the middle of the first plate and the second plate. The visual focusing point can be made by the screen-printing technique.

Since the plates are transparent and the pattern (preferably tarnished) is light enhanced to be clearly visible, the brightness of the light itself can produce a required contrast. Preferably, the light source (i.e. the first light source and the second light source), the opaque background board and the visual focusing element have colour contrast between each other. Herein the contrast as such is used as a general term but, it can be quantified and described in a more precise way via Colour Contrast Analysis Tools (for example [https://dequeuniversity.com/rules/axe/3.5/color-contrast]). Preferably, the light source is white, the opaque background board is black and the visual focusing element is red, in accordance with ISCEV standards [http://www.iscev.org]. As an example:
- a contrast ratio between white, background (lightness 100%, RGB 255,255,255) and red, foreground (lightness 50%, RGB 255,0,0) is 3.99:1;
- a contrast ratio between black, background (lightness 0%, RGB 0,0,0) and red, foreground (lightness 50%, RGB 255,0,0) is 5.25:1;
- a contrast ratio between white, background (lightness 100%, RGB 255,255,255) and black, foreground (lightness 0%, RGB 0,0,0) is 21:1.

The light source controlling means can be functionally divided into systems that serve defined and required purposes. The light source controlling means in the display of the invention comprises a triggering system, a light supply system and a brightness adjustment system. The light source controlling means including the triggering system, the light supply system and the brightness adjustment system can be any type of a system, a circuit or a computer logic or a program that would allow one to control the light sources in a desired manner. The light source controlling means of the display according to the invention provides:
- triggering of the illumination of the lights sources, preferably with selected, predetermined and/or adjustable frequency, and the illumination time delay adjustment of the light sources, operated by the triggering system;
- alternate illumination of the first light source and the second light source such that the light is supplied only to all of the first plate edges or to all of the second plate edges at a time, operated by the light supply system;
- brightness adjustment of the individual light sources, i.e. the first light source and the second light source, which are distributed along and optically coupled to the first plate edges and the second plate edges, respectively, operated by the brightness adjustment system.

Preferably, a brightness of the light sources, as measured for the single rectangular field of the checkerboard pattern, which may be defined as a unit of the first pattern or a unit of the second pattern, is in the range of 200 to 240 1x, more preferably 220 1x.

The display is especially intended for the examination of visual evoked potentials with simultaneous MRI acquisition. But the display according to the invention can also be used as a stand-alone element for use as a visual stimulus. Therefore the triggering system may be adapted accordingly and may comprise various elements and subsystems. The triggering system may cooperate with external actuation system and/or may comprise internal actuation subsystem. Additionally the triggering system comprises means to adjust illumination frequency of the lights sources, using an external system and/or an internal subsystem. The triggering system may comprise any suitable means to trigger the light sources or any suitable means to correlate a patient activity and the light sources and the display response, for example voice recognition subsystems.

Further, in order to assure required display reliability and dynamic characteristics, as well as a precise time locking, the time delay, i.e. the time between triggering signal and the display response (which is equivalent to the display board and display screen response, and in fact indicates the light source response of the display), should be in the range of milliseconds, preferably in the range of 1 to 20 milliseconds, more preferably to 10 milliseconds.

The display according to the invention may be part of a system for use in the examination of visual evoked potentials with simultaneous MRI. The system further comprises a reflecting means for suppling an image from the display to the patient 's eye and a system control means. The reflecting means can be an element of any type or a device that is unsusceptible to electromagnetic fields, preferably a tillable or pilotable mirror that can be located inside MRI bore, more preferably close to the patient's eye. The system control means are any means that allow one to control the system comprising the above-mentioned elements, for example, a synchronised MRI and the display operation, etc.

### BRIEF DESCRIPTION OF THE DRAWINGS

Embodiments of the invention are further described hereinafter with reference to the accompanying drawings, in which:
- FIG. 1A: shows an exploded view of a display screen comprising a display board and a light source;
- FIG. 1B: shows the/an assembled display screen;
- FIG. 2: shows a system according to the invention;
- FIG. 3A: shows time delay between a trigger signal and a display response for a commercial VEP CRT display;
- FIG. 3B: shows time delay between a trigger signal and a display response for a display according to the invention;
- FIG. 4 A, B: shows VEP measured parameters obtained with a display according to the invention used as a visual stimulus;
- FIG. 4 C, D: shows VEP measured parameters obtained with a commercial VEP CRT display used as a visual stimulus.

The figures are illustrative of the invention and have been made without precisely keeping to the scale and aspect ratio of the elements depicted therein.

### EMBODIMENTS OF THE INVENTION

### Display

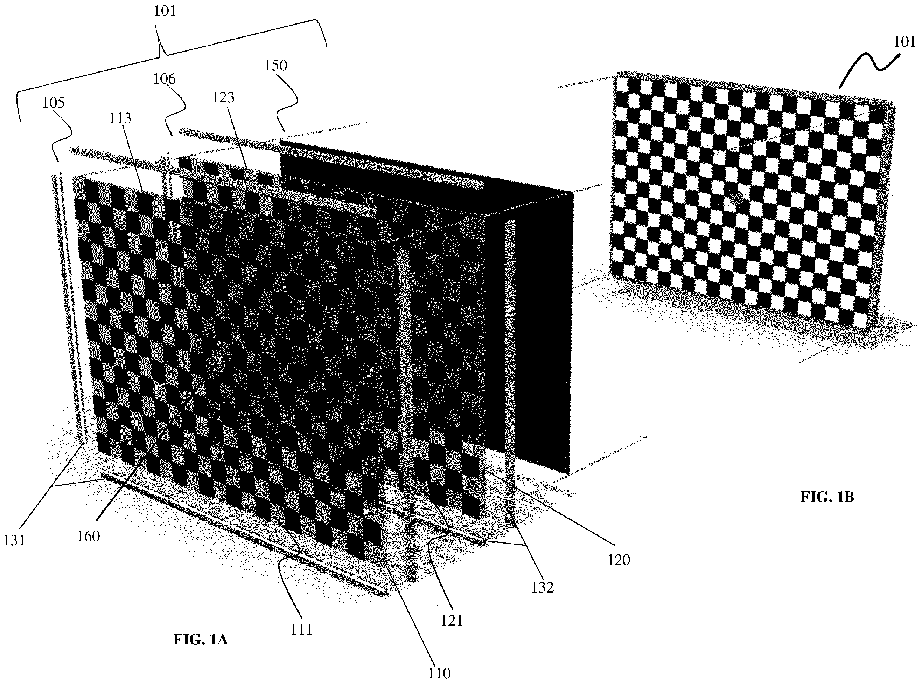

The exemplary embodiment of a display 100 according to the invention is provided below. The display 100 comprises a display screen 101, presented in FIG. 1A and FIG. 1B, and a light source controlling means 140 (not shown). The display screen 101 and the light source controlling means 140 are connected by a 12 m long screened cable 170. The display screen 101 comprises two display boards i.e. the first display board 105 and the second display board 106 and the opaque background board 150. The display screen 101 is shown in Fig. 1A and B, where FIG. 1A shows exploded view of the display screen 101, and FIG. 1B shows the assembled display screen 101.

### Display boards and the opaque background board

In one embodiment of the invention, as shown in FIG. 1 A as an exploded view of the display screen 101, the first display board 105 consists of the first plate 110 and the first light source 131, and the second display board 106 consists of the second plate 120 and the second light source 132. The first plate 110 and the second plate 120 are made of polished PMMA plates and the opaque background board 150 is made of a black, non-transparent plate.

A pattern is applied on the plates, i.e. on the first plate 110 and on the second plate 120. The first plate pattern 111 is applied to the first major surface of the first plate 110 and the second plate pattern 121 is applied to the first major surface of the second plate 120. Both the first plate pattern 111 and the second plate pattern 121 are checkerboard patterns. Both the first plate pattern 111 and the second plate pattern 121 are applied by engraving method by means of a milling machine (CNC machine), as a result of which alternated tarnished and polished rectangular fields forming the checkerboard pattern are obtained. For clarity reasons, the tarnished rectangular fields are depicted as white/grey and the transparent as black in FIG. 1A.

The checkerboard pattern applied on the first plate 110 is complementary to the checkerboard pattern on the second plate 120. It means that, when the first plate 110 is stacked on the second plate 120, the tarnished rectangular fields of the first plate 110 overlie on the polished rectangular fields of the second plate 120 and the polished rectangular fields of the first plate 110 overlie on the tarnished rectangular fields of the second plate 120, leading to substantially tarnished plane of the complementary first plate pattern 111 and the second plate pattern 121.

Fig. 1B shows the assembled display screen 101, and in a state when one light source is illuminated, showing a white glowing pattern of one plate and black, not glowing pattern of another plate.

The checkerboard patterns applied on the plates are in accordance with ISCEV standards [http://www.iscev.org].

In one embodiment the checkerboard pattern is applied on the plates of major surface dimensions 40 cm by 30 cm and comprises 20 by 14 rectangular fields, respectively.

In another embodiment the checkerboard pattern is applied on the plates of major surface dimensions 39 cm by 29 cm and comprises 78 by 58 rectangular fields, respectively.

The dimensions of the plates are not restricted as such, and can be selected as desired for a certain purpose, or defined by standards in certain fields. For example, the dimensions of the plates can be selected so as to obtain angular diameter (called also visual angle). The thickness of the plate depends on the material selected and the size of the light sources, and should ensure mechanical rigidity and stability, but on the other hand too thick plates may not assure sufficient pattern matching on both the first and second plates when stacked together.

In one embodiment a visual focussing element 160 is applied in the middle of both the first major surface of the first plate 110 and the first major surface of the second plate 120. The visual focussing element is a red dot of about 7 mm in diameter, applied by screen printing.

### Light source

In one embodiment both the first light source 131 and the second light source 132 comprise evenly distributed LED diodes (12V) arranged on all of the first plate edges 113 and all of the second plate edges 123, respectively, and they supply the light to the plates via the polished edges of the plates.

The first light source 131 arranged on the first plate edges 113 and the second light source 132 arranged on the second plate edges 123 are connected independently via the screened cable 170 to the light source controlling means 140. The screened cable 170 is 12 m long to allow the light source controlling means 140 of the display 100 to be located out of the electromagnetic fields range.

### Light source controlling means:

The light source controlling means was made on the basis of a programming platform for embedded systems Arduino, intended for microcontrollers mounted on a single printed circuit, with built-in support for input/output circuits and a standardized programming language. The microcontroller used is an Arduino Uno Rev3 with an AVR ATmega328 microcontroller.

The light source controlling means may be defined herein as comprising a triggering system, a light supply system and a brightness adjustment system.

### 1. Triggering system

In one embodiment, every half second an external TTL signal (trigger) arrives to Arduino, in the form of a single 5V peak lasting for fractions of a millisecond. After receiving the TTL trigger signal, it is necessary to change, in the same time, the state on the Arduino D12 output to high and Arduino D13 output to low with a strictly defined time delay, e.g. 10 milliseconds. The time delay is a time span that is applied between the trigger signal and the display response. After receiving the next signal, the state of D12 and D13 outputs should be inverted, i.e. into low at D12 output and high at D13 output, because after each TTL signal from the trigger there should be a change from the first light source actuation to the second light source actuation. After another trigger, again swap of the light source actuation should appear and so on.

In one embodiment the triggering system provides 2 Hz rectangular signal.

The Arduino operating voltage is 5V. This means that the power supply of the integrated circuits and their logical states now correspond to this voltage. This means that for the D12 and D13 outputs, regardless of the TTL trigger voltage (which here is also 5V), 5V alternately is obtained.

The time delay may be set for e.g. 10 milliseconds and is programmable. The value of the time delay parameter is extremely important, since it allows one to adjust the display 100 according to the invention to the commercial systems that are already in use for VEP measurements.

### 2. Light supply system

Since the voltage at the D12 and D13 outputs is 5V, it is insufficient to power the LEDs illuminating the display boards 105, 106, which require 12V. To increase the voltage, a transistor key was used, which in this case is a bipolar transistor .

In one embodiment the transistor key is a PNP transistor.

In another embodiment the transistor key is a NPN transistor.

The transistor key acts here as a voltage amplifier and as an "ON/OF" switch for the light sources. In one embodiment the display board is connected to two circuits with a NPN transistor, which are connected to the D12 and D13 outputs. In another embodiment the display board is connected to two circuits with a PNP transistor, which are connected to the D12 and D13 outputs.

### 3. Brightness adjustment system

Since the display screen 101 comprises two matted plates stacked one behind the other, it is necessary to adjust the brightness of both of them so as to provide the impression of the same brightness when the checkerboard flashes. For this purpose, the light source controlling means comprises a brightness adjustment system.

According to one embodiment the brightness adjustment system is a separate voltage dimming system, which is used after each transistor key of the triggering system. The brightness adjustment system comprises a potentiometer that can change and adjust the 12V voltage which is supplied to LEDs of the light sources 131, 132 by the light source controlling means, more precisely by the light supply system.

### System

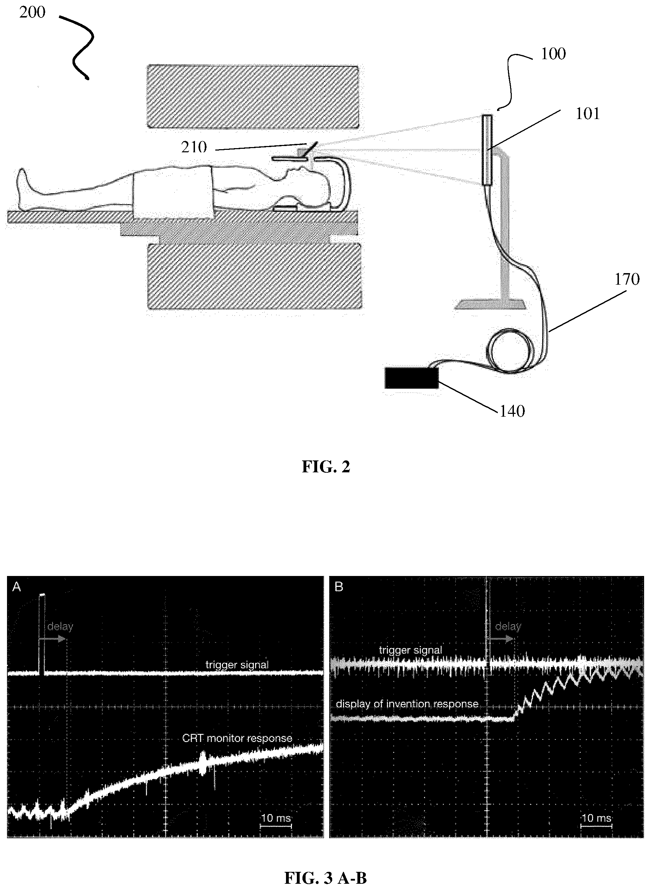

In another embodiment a system 200 for usage in the examination of visual evoked potentials with simultaneous MRI acquisition is presented as illustrated in FIG. 2. The system comprises the display 100 and the reflecting means 210 for suppling an image from the display 100 to the patient's eye. The reflecting means 210 are placed inside the MRI bore in the vicinity of the patient's eye, and in this embodiment it is a mirror that can be adjusted (tilted) in order to provide a visual stimulus from the display screen 101 to the patient's eye. Although the light source controlling means 140 are shown in FIG. 2, it must be pointed out that they are located out of the electromagnetic fields range and are connected to the display screen by the cable 170 that is unsusceptible to the electromagnetic fields, i.e. a screened cable.

### Comparative example

A display for VEP measurements has to be checked for correct time locking. FIG. 3A and 3B show that time delay of the display according to the invention was electronically set for the same values as a commercial VEP CRT display, compatible with ISCEV standards. Time delay is shown on the oscilloscope screen shoots by the arrow in Fig. 3A (the CRT monitor) and 3B (the display of the invention).

A group of patients were examined on commercial VEP apparatus and on the display 100 of the invention, as described in the exemplary embodiment. Latencies and amplitudes of N75, P100 and N135 peaks were measured. Then, two sets of data were compared, one obtained on the commercial VEP CRT display and the second set obtained on the display of the invention. The problem with comparison is that in a typical VEP measurement technique any result of measurement has relatively large uncertainty.

For the sake of simplicity each of the mentioned parameters is represented herein by v (thus, v stands for either latency or amplitude). The parameter v shows a statistical dispersion of the values attributed to a measured quantity. It may be safely assumed that for a patient in a stable health condition, the dispersion of the parameter v (i.e. its experimental uncertainty or standard deviation σ) is the result of influence of relatively large statistical noise on small in amplitude evoked potentials appearing on the electrodes. As the measurements of all patients were performed in the same conditions (e.g. the examination room, the siting place, the apparatus), the standard deviation σ is expected to be the same for all patients. Suppose that two independent measurements of parameter v are performed on each patient and they result in values v1, v2, and the values are shown as coordinates of point on the Cartesian frame. Data representation obtained this way corresponds to the well-known Deming regression, in which the uncertainties for the two variables are assumed to be independent and their standard deviations are the same. A great advantage of the Deming regression formalism is the possibility of determining σ having measured v1, v2 for a large enough group of patients. Deming regression was widely used in chemistry [Lin98], physiology [Bra77] and medicine [Haw02].

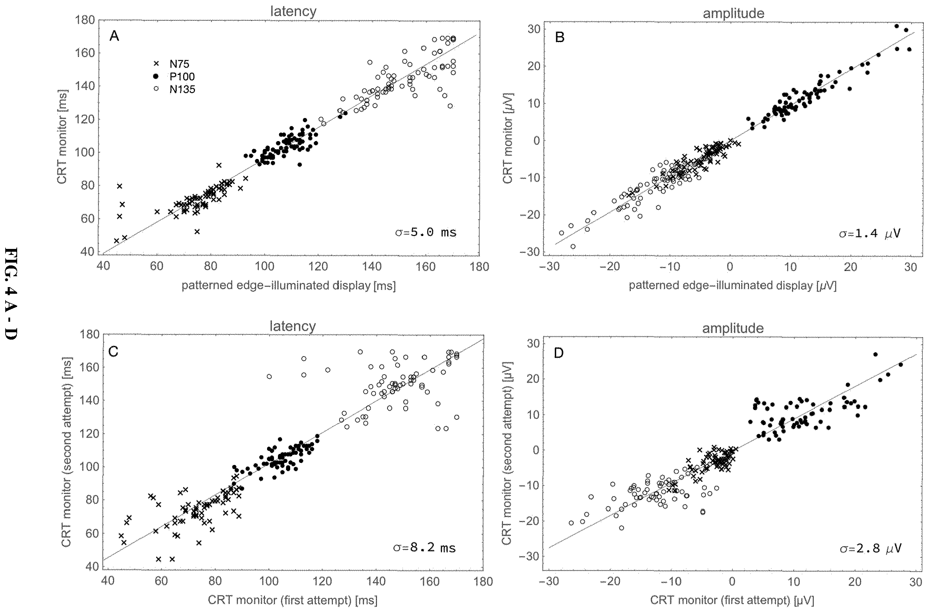

The results of the Deming correlation analysis of the results obtained on a commercial VEP CRT display and on the display of the invention is shown in Fig. 4 (A-D). It is clear that the results obtained on the commercial VEP CRT display (Fig 4 C, D) and on the display 100 of the invention (Fig 4 A,B), within the statistical uncertainties, are identical. The same range of correlation parameters was obtained for a group of patients investigated twice on the CRT monitor (Fig 4 C, D). Moreover, statistical uncertainties σ are indicated on each panel of Fig. 4. These values may serve as the uncertainty of each parameter v measured in clinical practice.

As it was demonstrated above, the display according to the invention, due to its structural and technical features, may be used in the vicinity of electromagnetic fields. It was shown that the performance of the display is excellent and exactly the same quality of visual stimuli may be provided by the display according to the invention. Moreover, the display is less complex structurally, and therefore easy to maintain apply and manufacture, but at the same time demonstrates required display reliability and dynamic characteristics, as well as a precise time locking.

### Final remarks:

Wherever e.g. (a/the) plate(s), (a/the) pattern(s), (a/the) light source(s), a/the major plane(s), (a/the) edge(s) are used herein, it should be understood as reference to all elements that are indicated and comprise such expressions, that is: (a/the) plate(s) comprise the first plate, the second plate, etc. with any added adjective.

While the foregoing description of the present invention has been presented with reference to particular embodiments and uses thereof, it is provided for the illustrative purposes and is not intended to be exhaustive or to limit the invention to specific embodiments and uses. It will be apparent to a person skilled in the art that a number of changes, modifications, or variations to the invention can be made as described herein, none of which depart from the spirit or scope of the present invention. The particular embodiments and applications have been selected and described to provide the best illustration of the principles of the invention and its practical application, thereby allowing a person skilled in the art to use the invention in various embodiments and with various modifications thereof. All such alterations, modifications, variations and modifications should therefore be regarded as falling within the scope of the present invention as defined in the appended claims when interpreted in accordance with the description.

### APPLICATION OF THE INVENTION

The invention can be used in many applications, but it is especially designed as a tool for medical examination, in particular the examination of visual evoked potentials. However, the invention may also be used in any application that requires well defined sequences of patterns to be displayed. The main advantage of the invention are structural features that make the display substantially unsusceptible to the electromagnetic fields. Therefore, the invention can be effectively used for the examination of visual evoked potentials with simultaneous MR imaging.

### LIST OF REFERENCE DESIGNATIONS

- 100: display

- 101: display screen

- 105: display board
- 106: display board

- 110: first plate
- 111: first plate pattern
- 113: first plate edges

- 120: second plate
- 121: second plate pattern
- 123: second plate edges

- 131: first light source
- 132: second light source

- 140: light source controlling means

- 150: opaque background screen

- 160: visual focusing element

- 170: cable

- 200: system
- 210: reflecting means

### Literature references:

[Arr13] J. Arrubla, I. Neuner, D. Hahn, F. Boers, N. J. Shah, Recording Visual Evoked Potentials and Auditory Evoked P300 at 9.4T Static Magnetic Field. PLoS ONE 8 (2013) e62915
[Bec05] R. Becker, P. Ritter, M. Moosmann and A. Villringer, Visual Evoked Potentials Recovered From fMRI Scan Periods, Human Brain Mapping 26:221-230(2005)
[Bon01] G. Bonmassar,1 D. P. Schwartz, A. K. Liu, K. K. Kwong, A. M. Dale, and J. W. Belliveau, Spatiotemporal Brain Imaging of Visual-Evoked Activity Using Interleaved EEG and fMRI Recordings, Neurolmage 13 (2001) 1035
[Bon01] G. Bonmassar,1 D. P. Schwartz, A. K. Liu, K. K. Kwong, A. M. Dale, and J. W. Belliveau, Spatiotemporal Brain Imaging of Visual-Evoked Activity Using Interleaved EEG and fMRI Recordings, Neurolmage 13 (2001) 1035
[Bra77] R. A. Brace, "Fitting Straight Lines to Experimental Data," The American Journal of Physiology, 233 (1977) 94
[Haw02] D. M. Hawkins, Diagnostics for conformity of paired quantitative measurements, Statist. Med. 21 (2002) 1913
[Hen05] S. Henning, K.-D. Merboldt and J. Frahm, Simultaneous recordings of visual evoked potentials and BOLD MRI activations in response to visual motion processing, NMR Biomed. 2005;18:543-552
[Huk02] A. C. Huk, R. F. Dougherty, and D. J. Heeger, Retinotopy and Functional Subdivision of Human Areas MT and MST,The Journal of Neuroscience, 22 (2002) 7195
[Kas01] K. Kashikura, J. Kershaw, S. Yamamoto, X. Zhang, T. Matsuura, I. Kanno, Temporal characteristics of event-related BOLD response and visual-evoked potentials from checkerboard stimulation of human V1: a comparison between different control features, Magn. Reson. Med. 45 (2001) 212
[Ko18] Y. Ko, S. D. Yun, S. Hong, Y. Ha, C. Choi, N. J. Shah, J. Felder, MR-compatible, 3.8 inch dual organic lightemitting diode (OLED) in-bore display for functional MRI, PLoS ONE 13 (2018) e0205325
[Lin98] K. Linnet, Performance of Deming regression analysis in case of misspecified analytical error ratio in method comparison studies, Clinical Chemistry 44 (1998) 1024
[Luo19] J. J. Luo, F. Bumanlag, N. Dun, Low-contrast visual evoked potential and early detection of optic demyelination, Journal of the Neurological Sciences 399 (2019) 108
[Mar20] F. A. Marie, I. Sinziana, I. Raluca, T. Ruxandra, C. Radu, V. Liliana, Visual evoked potential in the early diagnosis of glaucoma, Romanian Journal of Ophthalmology, 64 (2020) 15
[Nor15] A. M. Norcia, L. G. Appelbaum, J. M. Ales, B. R. Cottereau, & B. Rossion, The steady-state visual evoked potential in vision research: A review, Journal of Vision 15, (2015) 1
[Odo16] J. V. Odom . M. Bach . M. Brigell, G. E. Holder, D. L. McCulloch, A. Mizota , A. P. Tormene, ISCEV standard for clinical visual evoked potentials: (2016 update), Doc Ophthalmol. 133 (2016) 1
[Too14] A. T. Toosy, D. F. Mason, D. H. Miller, Optic neuritis, Lancet Neurol 13 (2014) 83

## Claims

1. A patterned edge-illuminated display (100) for use in the examination of visual evoked potentials with simultaneous MRI acquisition, comprising:
at least two plates, a first plate (110) and a second plate (120), wherein the first plate (110) and the second plate (120) are transparent, internally reflective light transmitting plates, each of the first plate (110) and the second plate (120) has two flat major surfaces parallel to each other, and edges;
a first light source (131) and a second light source (132) distributed along and optically coupled to first plate edges (113) and second plate edges (123), respectively;
a light source controlling means (140);
a first plate pattern (111) applied to at least the first major surface of the first plate (110) and a second plate pattern (121) applied to at least the first major surface of the second plate (120), which are configured to extract light from the first plate (110) and the second plate (120), respectively;
**characterised in that**
the display further comprises an opaque background board (150);
wherein the first plate (110) and the second plate (120) are stacked on the opaque background board (150), all with edges juxtapositioned to a respective edges of each other forming a display board (105);
the first plate pattern (111) is complementary to the second plate pattern (121); and
the first light source (131) is distributed along all of the first plate edges (113) and the second light source (132) is distributed along all of the second plate edges (123) and the light source controlling means (140) alternately illuminates the first light source (131) and the second light source (132) such that the light is supplied only to all of the first plate edges (113) or to all of the second plate edges (123) at a time.

2. The display (100) according to claim 1, **characterised in that** the first plate pattern (111) and the second plate pattern (121) are formed by tarnished, engraved parts of the first major surface of the first plate (110) and the first major surface of the second plate (120).

3. The display (100) according to claim 1 or 2, **characterised in that** the first plate pattern (111) and the second plate pattern (121) are checkerboard patterns.

4. The display (100) according to any of the preceding claims, **characterised in that** a visual focusing element (160) is arranged in the middle of the first plate (110) and the second plate (120).

5. The display (100) according to any of the preceding claims, **characterised in that** the first plate (110) and the second plate (120) are made from colourless material.

6. The display (100) according to any of the preceding claims, **characterised in that** the first and the second light sources (131, 132), the opaque background board (150) and the visual focusing element (160) have colour contrast between each other.

7. The display (100) according to any of the preceding claims, **characterised in that** the first plate (110), the second plate (120) and the background screen (150) are rectangular with the aspect ratio 4:3.

8. The display (100) according to any of the preceding claims, **characterised in that** the light source controlling means (140) comprises a triggering system which triggers operation of the first light source (131) and/or the second light source (132) and provides the first light source (131) and/or the second light source (132) delay adjustment.

9. The display (100) according to any of the preceding claims, **characterised in that** the light source controlling means (140) alternately illuminates the first light source (131) and the second light source (132) using rectangular signal of 2Hz frequency.

10. The display (100) according to any of the preceding claims, **characterised in that** the light source controlling means (140) comprises a brightness adjustment system which adjust separately the brightness of the first light source (131) distributed along and optically coupled to the first plate edges (113) and the second light source (132) distributed along and optically coupled to the second plate edges (113).

11. The display (100) according to any of the preceding claims, **characterised in that** the first plate pattern (111) and the second plate pattern (121) are checkerboard patterns having the dimensions of 40 cm by 30 cm divided into 20 by 14 rectangular fields or the dimensions of 39 cm by 29 cm divided into 78 by 58 rectangular fields.

12. The display (100) according to any of the preceding claims, **characterised in that** the visual focusing element (160) is a dot of 7 mm in diameter positioned at corners of the four rectangular fields of the checkerboard pattern, which are located in the middle of the first plate (110) and the second plate (120).

13. A system (200) for use in the examination of visual evoked potentials with simultaneous MRI acquisition, **characterised in that** it comprises:
- a patterned edge-illuminated display (100) according to claims 1 to 12;
- reflecting means (210) for suppling an image from the display screen (101) to the patient's eye;
- system control means.
